# NOUVEAU FASCICULE DE BREVET EUROPEEN

(11) **EP 0 526 678 B2**
(45) Date de publication et mention de la décision concernant l'opposition: **22.03.2000**
(45) Mention de la délivrance du brevet: 18.09.1996
(21) Numéro de dépôt: 91440110.4
(22) Date de dépôt: 27.12.1991
(51) Int. Cl.: A61J 1/14, A61M 5/165

(54) **Poche filtrante destinée à permettre la filtration stérile du sang et ensemble de poches de prélèvement du sang**
Filtrierende Beutel zur sterilen Filtration von Blut und Satz von Beuteln zum Entnehmen von Blut
Sterilising bag for sterile filtration of blood and set of bags for collection of blood

(30) Priorité: 24.06.1991 FR 9108262
(43) Date de publication de la demande: 10.02.1993
(73) Titulaire: MACO PHARMA, F-59200 Tourcoing (Nord) (FR)
(72) Inventeur: Goudaliez, Francis, F-59155 Faches-Thumesnil, (Nord) (FR)
(74) Mandataire: Derambure, Christian

(56) Documents cités:
- EP-A- 0 349 188
- WO-A-90/15660
- DE-A- 1 816 972
- GB-A- 1 517 731
- US-A- 4 066 556
- US-A- 4 596 657
- US-A- 4 810 378
- US-A- 4 997 577

## Description

L'invention est relative à une poche filtrante destinée à permettre la filtration stérile du sang total ou des concentrés de globules rouges pour en retenir les globules blancs, autrement appelés leucocytes, ainsi qu'à un ensemble de poches de prélèvement de sang utilisant la dite poche filtrante. Elle intéresse les fabricants de matériel médical.

Les besoins en composés sanguins labiles dépourvus de leucocytes sont de plus en plus importants en milieu hospitalier. Ils sont liés aux développements techniques qui permettent d'améliorer les résultats et d'en déduire de précieux bénéfices pour l'état de santé des individus transfusés.

A l'origine, le matériel qui était utilisé pour effectuer les prélèvements de sang se composait principalement de bocaux en verre réutilisables après stérilisation. Les progrès accomplis dans le domaine des matières plastiques ont permis de remplacer ces bocaux par des poches souples à usage unique. Outre, la plus grande commodité d'utilisation apportée par les poches souples, celles-ci permettent en plus, grâce aux systèmes de poches multiples, d'assurer la séparation des dérivés labiles du sang en circuit clos, sans risque de contamination septique.

Actuellement, les prélèvements sanguins sont réalisés à l'aide de poches stériles, confectionnées à l'aide de feuilles de matière plastique souple soudées mutuellement sur leur périphérie. Les poches sont reliées à une aiguille de prélèvement par l'intermédiaire d'une tubulure souple et, dans le cas de poches multiples, aux autres poches secondaires par l'intermédiaire également de tubulures souples.

Par centrifugation ou décantation, il est possible de séparer dans la poche de prélèvement les différents composés habiles du sang prélevé, à savoir le plasma, les globules blancs et les globules rouges qui se répartissent en couches. Il est alors judicieux de distribuer ces différents constituants du sang préalablement séparés dans des poches secondaires reliées à la poche de prélèvement. Les techniques actuelles sont parfaitement adaptées à ce genre de séparation stérile en circuit clos.

Lorsqu'il s'agit d'opérer une déleucocytation, c'est-à-dire lorsqu'il est nécessaire d'enlever d'un composant sanguin labile les globules blancs potentiellement ou assurément délétères pour le patient transfusé, l'emploi d'un filtre spécifique est une technique communément utilisée.

Pour cette opération, on relie la poche contenant le composant sanguin devant être débarrassé de leucocytes à une poche secondaire de recueil du composant dépourvu de leucocytes par l'intermédiaire d'un filtre.

Les filtres actuellement disponibles se présentant sous la forme d'un boîtier rigide dans lequel est intégré un milieu filtrant et muni d'une tubulure d'entrée terminée par un perforateur destiné à assurer la connexion avec la poche contenant le composant devant être filtré.

Par leur nature, ces boîtiers rigides ne peuvent être stérilisés à la vapeur et leur utilisation en centrifugation est difficile.

De plus, ces boîtiers filtrant rigides contiennent une quantité d'air qu'il sera nécessaire d'évacuer en fin de filtration par une tubulure parallèle en by-pass pour contourner le filtre rendu imperméable à l'air par l'imbibition du milieu filtrant.

Par ailleurs, on connaît du document GB-A-1.517.731 ou du document US-A-4.066.566 la structure d'une poche flexible constituée d'une enveloppe extérieure souple formée de feuilles assemblées sur leur périphérie et renfermant un filtre flexible dont les parties latérales sont scellées à un organe de support pour le solidariser dans la poche.

Cependant, ces deux documents concernent des domaines de filtration différents dans des circuits ouverts.

En effet, le filtre du document britannique permet de retenir des agrégats ou des substances étrangères lors de la transfusion du sang, en circuit ouvert, lors d'une opération chirurgicale ou d'une dialyse artificielle.

Le filtre du dispositif américain est quant à lui, non pas prévu pour la filtration du sang, mais utilisé pour le filtrage des fluides parentéraux pendant leur administration. Autrement dit, on est dans un système de perfusion en circuit ouvert et on cherche à éliminer les matières particulières pouvant se trouver dans les solutions parentérales à administrer.

Les buts poursuivis par ces documents sont différents et ne permettent pas d'arriver à la solution au problème posé par la filtration stérile du sang total et plus spécialement pour la déleucocytation.

Le but principal de la présente invention est de présenter une poche filtrante souple qui dispose des mêmes commodités d'utilisation que les poches souples de prélèvement et de transfert communément employées.

Ainsi, la poche filtrante de la présente invention peut parfaitement être stérilisée à la vapeur et être utilisée dans une centrifugeuse. Par ailleurs, la poche filtrante pourra être intégrée d'origine dans un système de poche mutiple pour réaliser des filtrations en circuit clos dans des conditions d'aseptie maximale puisque, à aucun moment, des branchements doivent être réalisés entre éléments séparés.

Un autre avantage de la présente invention est que la souplesse de la poche filtrante rend inutile l'évacuation de l'air résiduel par l'intermédiaire d'une tubulure parallèle en by-pass.

Les parois extérieures souples ont tendance à se coller sur le milieu filtrant, ce qui pourrait défavoriser le passage du liquide à filtrer. Une attention particulière a été apportée à ce problème potentiel qui a été résolu par l'interposition entre la paroi souple et le milieu filtrant de joncs de matière plastique souple.

Un autre avantage de la présente invention est la transparence des parois de la poche filtrante permettant à l'utilisateur une visualisation parfaite du stade de filtration et notamment de la fin du passage du liquide à filtrer.

D'autres buts et avantages de la présente invention apparaitront au cours de la description qui va suivre, qui n'est cependant donnée qu'à titre indicatif.

Selon l'invention, l'ensemble de poches souples pour prélèvement de sang et autorisant la déleucocytation comprend au moins une poche principale de prélèvement, reliée à une poche secondaire de recueil, via une poche filtrante, ainsi qu'un ensemble de tubulures souples, ladite poche filtrante comprenant une enveloppe extérieure souple formée de deux feuilles de matière plastique souple assemblées sur leur périphérie renfermant un milieu filtrant, qui est maintenu dans un cadre souple étanche qui délimite deux compartiments respectivement d'entrée et de sortie de la poche filtrante, raccordés par une desdites tubulures respectivement à la poche principale et à la poche secondaire.

Par ailleurs, selon l'invention, la poche filtrante souple, destinée à permettre la filtration du sang ou des composants sanguins labiles pour en séparer les globules blancs, comprenant une enveloppe extérieure souple formée de deux feuilles de matière plastique souple assemblées sur leur périphérie renfermant un milieu filtrant, qui est maintenu dans un cadre souple étanche qui délimite deux compartiments respectivement d'entrée et de sortie de la poche filtrante, est caractérisée par le fait que le compartiment de sortie est dégagé du milieu filtrant par la présence de joncs d'écartement souples.

L'invention sera mieux comprise à la lecture de la description suivante accompagnée de dessins en annexe parmi lesquels :
- la figure 1 schématise un ensemble de poches destinées au prélèvement du sang, à sa séparation en différents composants labiles et à la filtration du concentré de globules rouges pour en assurer la déplétion en globules blancs,
- la figure 2 représente, en vue de coupe, une poche filtrante selon la présente invention,
- la figure 3 illustre la mise en place du milieu filtrant sur un cadre souple de support,
- la figure 4 montre la mise en place du milieu filtrant dans la poche, - la figure 5 montre la mise en place des joncs d'écartement des parois de la poche filtrante.

La présente invention vise une poche filtrante, destinée à permettre la déleucocytation d'un composant labile du sang ainsi qu'un ensemble de poches à prélèvement de sang utilisant la dite poche filtrante. Elle concerne principalement les fabricants de matériel et de produits médicaux.

L'objectif de la présente invention est une poche filtrante souple qui puisse parfaitement s'intégrer dans un système de poches souples primaires et secondaires de prélèvement et de séparation des composants labiles du sang. La poche filtrante de l'invention pourra ainsi subir des opérations de stérilisation à la vapeur et de centrifugation du système de poche. L'aseptie durant l'ensemble des opérations de séparation des composants du sang est assurée puisqu'à aucun moment il n'est pratiqué d'ouverture du circuit clos vers l'extérieur.

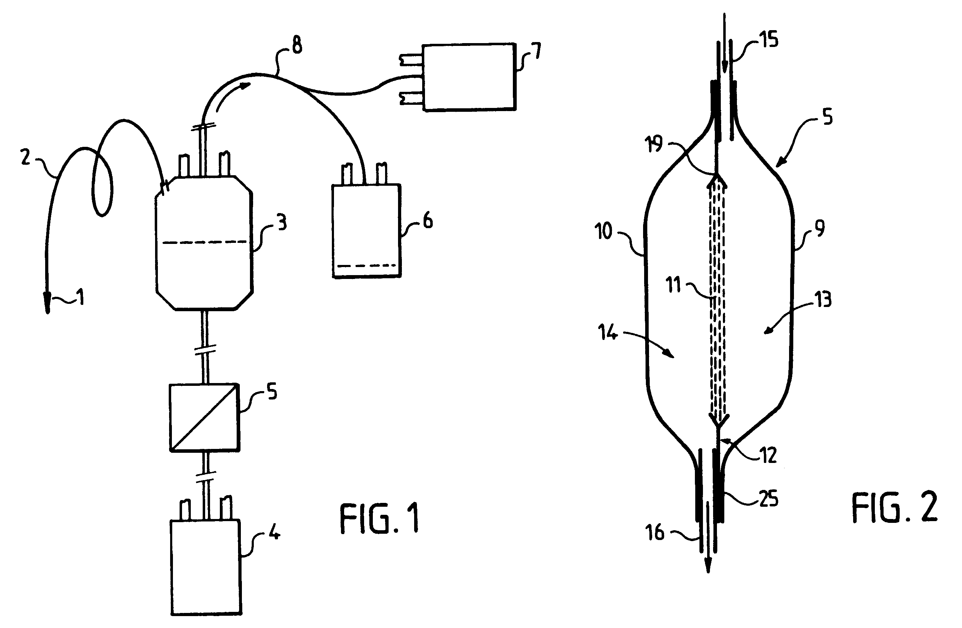

La figure 1 représente un système de poches plus spécialement adapté à une déplétion en leucocytes d'un concentré de globules rouges obtenu par séparation d'une unité de sang total. On retrouve une aiguille 1 de prélèvement, à introduire dans la veine du donneur, reliée par une tubulure souple 2 à une poche principale 3 souple dans laquelle le sang est recueilli. Cette poche principale 3 contenant une solution anticoagulante est reliée à une poche secondaire 4 de recueil des globules rouges via une poche filtrante 5 intermédiaire objet de la présente invention.

Par ailleurs, la poche principale 3 est par exemple aussi reliée à des poches secondaires 6 et 7 par l'intermédiaire d'un ensemble 8 de tuyaux souples vers lesquels sont dirigés respectivement le plasma riche en plaquettes et le plasma pauvre en plaquettes séparé par centrifugation selon des techniques connues de l'homme de l'art.

Bien entendu, la poche filtrante 5 souple de la présente invention pourra également avoir d'autres applications. Toutefois, ici on peut déjà en apprécier les avantages à savoir une possibilité de stérilisation globale de l'ensemble des poches à la vapeur et transfert des dérivés du sang sans ouverture du circuit à l'air libre. En outre, la poche filtrante peut parfaitement subir une centrifugation sans dommage pour elle-même ou les autres poches contenues en même temps dans le même pot de centrifugation.

La figure 2 schématise en vue de coupe longitudinale la poche filtrante 5 de l'invention. Celle-ci comporte une enveloppe extérieure souple formée d'un assemblage de deux feuilles de matière plastique 9 et 10 assemblées mutuellement sur leur périphérie. Cette enveloppe extérieure renferme un milieu filtrant 11 qui, selon l'invention, est maintenu dans un cadre 12 souple étanche qui délimite deux compartiments, respectivement d'entrée 13 et sortie 14 de la poche filtrante 5.

Le compartiment 13 communique avec l'extérieur de la poche 5 par l'intermédiaire d'une tubulure souple d'entrée 15 qui sert au remplissage de la poche filtrante. Le compartiment 4 communique avec l'extérieur de la poche 5 par l'intermédiaire d'une tubulure souple de sortie 16 qui assure l'évacuation du liquide filtré.

Ainsi, selon la présente invention, on résoud les difficultés d'étanchéïté en deux niveaux. Tout d'abord, on réalise une première étanchéïté entre le milieu filtrant 11 et le cadre souple 12 où il n'y a aucun passage de tubulure mais où il faut assurer une jonction entre des matériaux de natures différentes à savoir le milieu filtrant et un cadre plastifié 12. Ensuite, le deuxième niveau d'étanchéïté qu'il faut assurer se situe à la périphérie de la poche filtrante 5 où il faut assurer la jonction entre les feuilles extérieures 9 et 10, la périphérie du cadre 12 souple et le passage des tubulures souples d'entrée des liquides 15 et de sortie des liquides 16.

Ce second niveau d'étanchéïté à assurer peut être réalisé selon des techniques connues de l'homme de l'art à partir du moment où l'on fait appel, pour la conception de la poche, à des feuilles de matière plastique souples 9 et 10, ainsi que pour le cadre souple 12, la même matière plastique souple étant utilisée pour les tubulures d'entrée 15 et de sortie 16. Parmi les techniques connues on pourra par exemple retenir le soudage par haute fréquence.

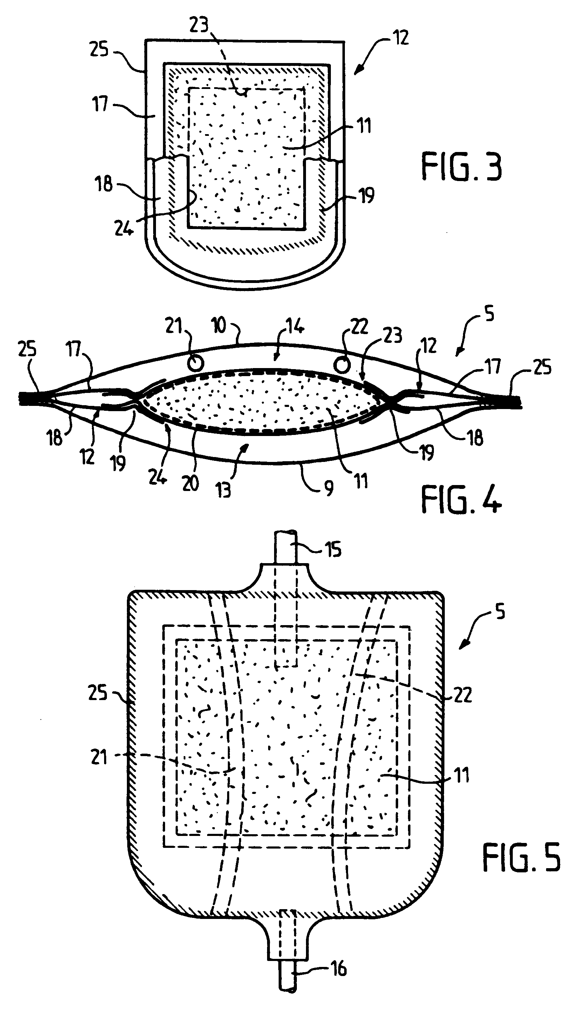

La figure 3 montre, en vue de coupe partielle, la conception du cadre souple 12 de support de milieu filtrant 11. Ce cadre 12 est formé par un assemblage de deux feuilles plastifiées 17 et 18 entre lesquelles le milieu filtrant 11 est placé. Ces deux feuilles 17 et 18 sont ajourées dans leur partie centrale et présentent chacune au moins une ouverture 23, 24 afin que le milieu filtrant 11 puisse communiquer de chaque côté.

Les feuilles 17 et 18 sont fixées entre elles au niveau de la périphérie du milieu filtrant 11, par exemple par un cordon de soudure 19. L'avantage d'utiliser un cordon de soudure 19 périphérique est qu'il assure non seulement la fixation du milieu filtrant 11 au centre du cadre souple 12, mais également qu'il forme un joint périphérique d'étanchéité qui évite que les liquides puissent s'écouler entre les feuilles 17 et 18.

La figure 4 représente en vue de coupe la poche filtrante souple 5 de la présente invention. On peut remarquer la soudure des feuilles 17 et 18 à travers le milieu filtrant 11 fortement comprimé à ce niveau pour former un cordon 19 étanche.

La périphérie 25 du cadre souple 12 est soudée avec les feuilles extérieures 9 et 10, mutuellement sur tout leur pourtour, qui forment l'enveloppe de la poche filtrante 5. L'étanchéïté est par conséquent également assurée à ce niveau.

Le milieu filtrant 11 pourra être confectionné par exemple par juxtaposition de couches filtrantes. Par exemple, le nombre de couches pourra varier de quatre à vingt. On pourra également avantageusement utiliser un préfiltre 20 en forme de grille ou de non-tissé à porosité supérieure sur l'extérieur du milieu filtrant 11 pour éviter que celui-ci ne se colmate.

Il a été remarqué que le milieu filtrant 11 peut avoir tendance a se coller sur la paroi extérieure 10 de l'enveloppe qui constitue le compartiment 14 de sortie. Ce phénomène gêne le passage des fluides

Pour pallier ce défaut, on place avantageusement, par exemple, deux joncs 21 et 22 à l'intérieur de la feuille 10 qui forme le compartiment 14 de sortie pour éviter que la couche extérieure du milieu filtrant 11 ne puisse venir se plaquer sur la paroi intérieure de la feuille 10.

En pratique, les joncs 21 et 22 sont constitués notamment par des parties de tubulures souples soudées au niveau de la paroi intérieure de la feuille 10, notamment au niveau de la soudure périphérique 25 de la poche filtrante 5, comme cela est illustré à la figure 5. Ces joncs 21 et 22 permettent d'écarter la feuille 10 du milieu filtrant 11. Par leur souplesse, les joncs 21 et 22 n'altèrent pas les propriétés de pliage de la poche filtrante 5.

D'autres buts et avantages de la présente invention, à la portée de l'homme de l'art, auraient également pu être envisagés sans pour autant sortir du cadre de celle-ci, telle que definie par les revendi cations suivantes.

## Revendications

1. Ensemble de poches souples pour prélèvement de sang et autorisant la déleucocytation en circuit clos, comprenant au moins une poche principale de prélèvement (3), reliée à une poche secondaire de recueil (4), via une poche filtrante (5), ainsi qu'un ensemble de tubulures souples (2, 15, 16), ladite poche filtrante (5) comprenant une enveloppe extérieure souple formée de deux feuilles (9, 10) de matière plastique souple assemblées sur leur périphérie (25) renfermant un milieu filtrant (11), qui est maintenu dans un cadre (12) souple étanche qui délimite deux compartiments (13, 14) respectivement d'entrée et de sortie de la poche filtrante (5), raccordés par une desdites tubulures (15, 16) respectivement à la poche principale (3) et à la poche secondaire (4).

2. Ensemble de poches selon la revendication 1, caractérisé par le fait que le cadre (12) est formé par un assemblage de deux feuilles (17 et 18) souples ajourées entre lesquelles le milieu filtrant (11) est placé.

3. Ensemble de poches selon la revendication 2, caractérisé par le fait que les feuilles (17, 18) qui forment le cadre (12) sont fixées entre elles sur la périphérie (19) du milieu filtrant (11) et également avec les feuilles (9, 10) au niveau de la périphérie (25) de l'enveloppe de la poche filtrante (5).

4. Ensemble de poches selon la revendication 3, caractérisé par le fait que la fixation des feuilles (17, 18) qui forment le cadre souple (12) est un cordon de soudure (19) réalisé à travers le milieu filtrant (11).

5. Ensemble de poches selon la revendication 1, caractérisé par le fait que le milieu filtrant (11) comporte un préfiltre (20) extérieur qui se présente sous la forme d'une grille.

6. Ensemble de poches selon la revendication 1, caractérisé par le fait que le compartiment de sortie (14) est dégagé du milieu filtrant (11) par la présence de joncs d'écartement (21 et 22) souples.

7. Ensemble de poche selon la revendication 6, caractérisé par le fait que les joncs d'écartement (21 et 22) souples sont constitués par des tubulures soudées au niveau de la paroi intérieure de la feuille (10) de la poche filtrante.

8. Ensemble de poche selon la revendication 1, caractérisé par le fait que la poche principale (3) est en outre reliée à d'autres poches secondaires (6, 7) par l'intermédiaire d'un ensemble (8) de tuyaux souples pour recevoir respectivement le plasma riche en plaquettes et le plasma pauvre en plaquettes séparés par centrifugation.

9. Poche filtrante souple (5), destinée à permettre la filtration du sang total ou des composants sanguins labiles pour en séparer les globules blancs, comprenant une enveloppe extérieure souple formée de deux feuilles (9, 10) de matière plastique souple assemblées sur leur périphérie (25) renfermant un milieu filtrant (11), qui est maintenu dans un cadre (12) souple étanche qui délimite deux compartiments (13, 14) respectivement d'entrée et de sortie de la poche filtrante (5), le compartiment de sortie (14) étant dégagé du milieu filtrant (11) par la présence de joncs d'écartement (21, 22) souples.

10. Poche filtrante selon la revendication 9, caractérisée par le fait que les joncs d'écartement (21, 22) souples sont constitués par des tubulures soudées au niveau de la paroi intérieure de la feuille (10) de la poche filtrante.

## Claims

1. Set of flexible bags for taking blood samples and permetting closed system deleucocytation, comprising at least a main sampling bag (3), connected to a secondary collecting bag (4), via filtering bag (5), as well as a set of flexible pipes (2, 15, 16), the said filtering bag (5) including a flexible outer envelope formed by two sheets (9, 10) of flexible plastic material assembled on their periphery (25) enclosing a filtering medium (11), which is held in a tight, flexible frame (12) which delimits two compartments (13, 14), an input compartment and an output compartment respectively, of the filtering bag (5), which compartments are connected by one of the said tubes (15, 16) respectively to the main bag (3) and to the secondary bag (4).

2. Set of bags according to claim1, characterized by the fact that the frame (12) is formed by an assembly of two flexible, openwork sheets (17 and 18) between which the filtering medium (11) is placed.

3. Set of bags according to claim 2, characterized by the fact that the sheets (17, 18) that form the frame (12) are fixed together on the periphery (19) of the filtering medium (11), as well as to the sheets (9, 10) in the area of the periphery (25) of the envelope of the filtering bag (5).

4. Set of bags according to claim 3, characterized by the fact that the means for fixing the sheets (17, 18) that form the flexible frame (12) is a weld seam (19) produced through the filtering medium (11).

5. Set of bags according to claim 1, characterized by the fact the filtering medium (11) comprises an outer pre-filter (20) which takes the form of a grid.

6. Set of bags according to claim 1, characterized by the fact that the output compartment (14) is kept clear of the filtering medium (11) by means of flexible spacing rods (21 and 22).

7. Set of bags according to claim 6, characterized by the fact that the flexible spacing rods (21 and 22) are formed by tubes welded in the area of the inner wall of the sheet (10) of the filtering bag.

8. Set of bags according to claim 1, characterized by the fact the main bag (3) is further connected to other secondary bags (6, 7) via a set (8) of flexible tubes for receiving respectively plasma that is rich in platelets and plasma that is poor in platelets separated by centrifuging.

9. Flexible filtering bag (5) designed to permit the filtering of the total blood or the able blood components for separating the white blood cells therefrom, including a flexible outer envelope formed by two sheets (9, 10) of flexible plastic material assembled on their periphery (25) enclosing a filtering medium (11), which is held in a tight, flexible frame (12) which delimits two compartments (13, 14), an input compartment and an output compartment respectively, of the filtering bag (5), the output compartiment being kept clear of the filtering medium (11) by means of flexible spacing rods (21, 22).

10. Filtering bag according to claim 9, charactherized by the fact that the flexible spacing rods (21, 22) are constituted by tubes welded in the area of the inner wall of the sheet (10) of the filtering bag.

## Patentansprüche

1. Biegsame Beuteleinheit zur Entnahme von Blut, die die Trennung von Leukozyten im geschlossenen System erlaubt, umfassende wenigstens einen Hauptentnahmebeutel (3), der über einen Filtrierbeutel (5) mit einem Sekundäraufnahmebeutel (4) verbunden ist, sowie einen Satz biegsamer Röhrchen (2, 15, 16), wobei der genannte Filtrierbeutel (5) eine biegsame Außenhülle umfaßt, die aus zwei an deren Umkreis (25) verbundenen Folien (9, 10) aus weichen Kunststoff gebildet ist und ein Filtriermedium (11) einschließt, das in einem dichten, biegsamen Rahmen (12) gehalten wird, der zwei Ein- bzw. Auslaßabteilungen (13, 14) des Filtrierbeutels (5) begrenzt, die über eines der genannten Rörchen (15, 16) respektive mit dem Hauptbeutel (3) und dem Sekundärbeutel (4) verbunden sind.

2. Beuteleinheit nach Anspruch 1, dadurch gekennzeichnet, daß der Rahmen (12) aus einer Verbindung von zwei durchlochten, weichen Folien (17 und 18) gebildet ist, zwischen die das Filtriermedium (11) angebracht wird.

3. Beuteleinheit nach Anspruch 2, dadurch gekennzeichnet, daß die den Rahmen (12) bildenden Folien (17, 18) am Umkreis (19) des Filtriermediums (11) an einander, und ebenfalls im Bereich des Umkreises (25) der Hülle des Filtrierbeutels (5) an den Folien (9, 10) befestigt sind.

4. Beuteleinheit nach Anspruch 3, dadurch gekennzeichnet, daß die Befestigung der den biegsamen Rahmen (12) bildenden Folien (17, 18) eine durch das Filtriermedium (11) hindurch hersgestellte Schweißnacht (19) ist.

5. Beuteleinheit nach Anspruch 1, dadurch gekennzeichnet, daß das Filtriermedium (11) einen als ein Gitter ausgestalteten, auswendingen Vorfilter (20) umfaßt.

6. Beuteleinheit nach Anspruch 1, dadurch gekennzeichnet, daß die Auslaßabteilung (14) durch das Vorhandensein biegsamer Abstandsröhrchen (21 und 22) vom Filtriermedium (11) beabstandet ist.

7. Beuteleinheit nach Anspruch 6, dadurch gekennzeichnet, daß die Abstandsröhrchen (21 und 22) aus im Bereich der Innenwand der Folie (10) des Filtrierbeutels geschweißten Rörchen bestehen.

8. Beuteleinheit nach Anspruch 1, dadurch gekennzeichnet, daß der Hauptbeutel (3) außerdem über einen Satz (8) biegsamer Röhrchen mit weiteren Sekundärbeuteln (6, 7) zur Aufnahme respektive des plättchenreichen Plasmas und des plättchenarmen Plasmas, die durch Schleudern von einander getrennt werden, verbunden ist.

9. Biegsamer Filtrierbeutel (5) zum Erlauben der Filtrierung des Gesamtblutes oder der labilen Blutkomponenten, zum Trennen der weißen Blutkörperchen, umfassende eine biegsame Außenhülle, die aus zwei an deren Umkreis (25) verbundenen Folien (9, 10) aus weichen Kunststoff gebildet ist und ein Filtriermedium (11) einschließt, das in einem dichten, biegsamen Rahmen (12) gehalten wird, der zwei Ein- bzw. Auslaßabteilungen (13, 14) des Filtrierbeutels (5) begrenzt, wobei die Auslaßabteilung (14) durch das Vorhandensein biegsamer Abstandsröhrchen (21, 22) vom Filtriermedium (11) beabstandet ist.

10. Filtrierbeutel nach Anspruch 9, dadurch gekennzeichnet, daß die Abstandsröhrchen (21, 22) aus im Bereich der Innenwand der Folie (10) des Filtrierbeutels geschweißten Rörchen bestehen.
